# EUROPÄISCHE PATENTSCHRIFT

(11) **EP 2 996 585 B1**
(45) Veröffentlichungstag und Bekanntmachung des Hinweises auf die Patenterteilung: **08.11.2017**
(21) Anmeldenummer: 14719244.7
(22) Anmeldetag: 12.03.2014
(51) Int. Cl.: A61B 17/28, A61B 17/29, A61B 17/16, A61B 17/88, A61B 90/00

(54) **CHIRURGISCHES INSTRUMENT**
SURGICAL INSTRUMENT
INSTRUMENT CHIRURGICAL

(30) Priorität: 15.03.2013 DE 102013102679
(43) Veröffentlichungstag der Anmeldung: 23.03.2016
(73) Patentinhaber: Karl Klappenecker GmbH & Co. KG, 78532 Tuttlingen-Nendingen (DE)
(72) Erfinder: SPITZNAGEL, Bernhard, 78606 Seitingen-Oberflacht (DE)
(74) Vertreter: Patentanwälte und Rechtsanwalt Weiß, Arat & Partner mbB
(86) Internationale Anmeldenummer: PCT/EP2014/054838
(87) Internationale Veröffentlichungsnummer: WO 2014/140100

(56) Entgegenhaltungen:
- EP-A2- 0 534 303
- DE-A1- 19 948 031
- DE-A1-102010 013 296
- US-A1- 2006 116 706

## Beschreibung

Die vorliegende Erfindung betrifft eine Vorrichtung gemäss dem Oberbegriff des Anspruchs 1.

### STAND DER TECHNIK

Aus dem Stand der Technik sind eine grosse Vielzahl chirurgischer Instrumente bekannt, beispielsweise Zangen, Spreizer, Klemmen, Bügel, Sperrer und Scheren in verschiedensten Ausführungsformen und für die unterschiedlichsten chirurgischen Anwendungen.

Chirurgische Instrumente sind bevorzugt ergonomisch geformt und von einem Operateur mit einer Hand bedienbar.

In diesem Zusammenhang wird auf die DE 199 48 03 A1 verwiesen, welche ein chirurgisches Rohrschaftinstrument, wobei die Kraftausbildung durch ein Griffelement auf das Werkzeug derart begrenzt ist, dass auch die Längsbewegung des eines Betätigungselements begrenzt ist.

Weiter wird auf die DE 10 2010 013 296 A1 hingewiesen, welche ein chirurgisches Instrument mit einer Verriegelungsnase und einer Feder offenbart, wobei die Verriegelungsnase und die Feder einstückig ausgebildet sind.

Daneben wird auf die EP 0 534 303 A2 und die US 2006/0116706 A1 verwiesen, welche ebenfalls verschiedene Schiebeschaftinstrumente nach dem Stand der Technik aufzeigen.

Oft weisen derartige chirurgische Instrumente eine Feder auf, so dass der Operateur die Bewegung des Instruments mit einem gewissen Kraftaufwand in eine Richtung vollführt, und die gegensätzliche Bewegung, also die Bewegung in eine Ausgangsposition zurück, durch eine Rückstellkraft der Feder bewerkstelligt wird.

Im Bereich chirurgischer Instrumente, vielmehr im Bereich medizinischer Vorrichtungen allgemein, stellt in jüngster Zeit die Reinigung von oft komplexen Vorrichtungen ein wachsendes Problem dar. Während die Vorrichtungen technisch immer ausgefeilter werden, bedarf es zu ihrer Reinigung eines immer grösseren Aufwands.

Insbesondere stellen hier Teilbereiche medizinischer Vorrichtungen, welche mittels eines Reinigungsprozesses, insbesondere unter Verwendung von Reinigungsmittel, nicht in einen sterilen Zustand versetzt werden können, ein immenses Risiko dar.

### AUFGABE

Die Aufgabe der vorliegenden Erfindung ist es, die Nachteile des Standes der Technik zu überwinden. Insbesondere soll eine Vorrichtung bereitgestellt werden, die einfach in Einzelteile zerlegbar und ebenso einfach zusammensetzbar ist und die auf einfache Weise in einem Reinigungsprozess derart gereinigt werden kann, dass sie steril ist.

### LÖSUNG DER AUFGABE

Zur Lösung der Aufgabe führen die Merkmale des Anspruchs 1.

Das erfindungsgemässe chirurgische Instrument ist vorzugsweise ein Schiebeschaftinstrument, das verschiedene Funktionselemente aufweisen kann. Das Funktionselement ist das mit dem Schiebeschaftinstrument betätigte Element, wobei besonders bevorzugt Zangen- oder Schneidelemente als Funktionselemente verwendet werden.

Ein Ausführungsbeispiel für eine erfindungsgemässes chirurgisches Instrument ist eine Litzeldrahtschneider.

Die Verbindung zwischen Schaft und Schiebeteil ist bevorzugt über Schienen oder Nuten gestaltet. Alls Führungen sind offen, es entstehen keinerlei Toträume oder spitze Ecken/Winkel. Auf diese Weise sind ferner keinerlei Fügestellen vorhanden. Bei dem erfindungsgemässen chirurgischen Instrumente bedarf es keinerlei Schrauben, Nieten, Schweissstellen oder dergleichen. Daher ist auch keine Korrosion zu befürchten.

Wichtig ist in diesem Zusammenhang, dass das Schiebeteil auf den Schaft zum Einen horizontal durch Bewegung eines zweiten Griff verschoben werden kann und zum Anderen das Schiebeteil von dem Schaft gelöst werden kann. Das Schiebeteil ist bevorzugt von hinten, d.h. proximal in den Schaft einschiebbar.

An dem Schaft ist ein erster Griff ausgeformt. Dieser erste Griff steht in Wirkverbindung mit dem zweiten Griff. Dabei ist der zweite Griff schwenkbar mit dem Schaft konnektiert. Weiter ist das Schiebeteil durch den zweiten Griff betätigbar. Das bedeutet, dass das Schiebeteil relativ zu dem Schaft durch Heranziehen des zweiten Griffs an den ersten Griff verschoben werden kann. Ausserdem ist ein Kraftspeicher zum Rückstellen des Schiebeteils and dem Schaft vorhanden. Das Rückstellen geschieht in der Weise, dass der zweite Griff in seine Ausgangsposition rückgestellt wird und durch manuelles Betätigen wieder zum ersten Griff gezogen werden kann oder muss. Vorteilhaft hierbei ist der Umstand, dass das chirurgische Instrument werkzeuglos zerlegbar und zusammensetzbar ist.

Die erfinderische Besonderheit liegt darin, dass der Kraftspeicher und das Schiebeteil einstückig ausgebildet sind. Die Form der einstückigen Ausbildung ist nur insofern beachtlich, als dass die gewählte Form ermöglichen muss, dass das Schiebeteil zu dem Schaft rückgestellt werden kann. Dazu bedarf es in der Regel einer Kontaktstelle des Kraftspeichers mit dem Schaft, wobei die Kontaktstelle als Kraftanker dient. Vorteilhaft hierbei wiederum ist der Umstand, dass die einstückige Ausbildung des Kraftspeichers mit dem Schaft dafür sorgt, dass keine toten Flächen vorhanden sind, was eine einfache Sterilisierung ermöglicht. Dies gerade vor dem Hintergrund, dass ansonsten eingesetzte Bauteile, wie beispielsweise Flachfedern, nur aufwendig sterilisiert werden können.

Weiterhin weist der zweite Griff einends einen Haltebereich auf. Dieser Haltebereich umfasst Fingermulden. Diese Fingermulden dienen der besseren und einfacheren Handhabbarkeit des chirurgischen Instruments. Sie erhöhen die Nutzerfreundlichkeit und die Sicherheit.

Der zweite Griff weist andernends eine Ausnehmung zur Koppelung mit dem Schiebeteil auf. Die Ausnehmung kann hierbei entweder in Form eines Lochs, einer Öffnung oder einer Mulde vorgesehen sein. Letztendlich kommt es nur darauf an, dass der zweite Griff eine schwenkbare Verbindung mit dem Schiebeteil eingehen kann.

Der zweite Griff bildet einen Einhängebolzen aus. Dieser Einhängebolzen dient der Koppelung mit dem Schaft. In einem bevorzugten Ausführungsbeispiel ist der Einhängebolzen unrund ausgeformt. Der Einhängebolzen soll eine ebenfalls schwenkbare Verbindung mit dem Schaft ermöglichen. Die unrunde Ausgestaltung des Einhängebolzens verhindert im Zusammenspiel mit einer Einhängeausnehmung des Schafts ein versehentliches Falsch-Einschieben des zweiten Griffs in den Schaft.

Weiter weist ein Ausführungsbeispiel im Bereich der Einhängeausnehmung einen Durchgriff in dem Schaft auf. Dieser Durchgriff ist derart ausgeformt, dass der zweite Griff derart in den Schaft hineinragt, dass die Ausnehmung des zweiten Griffes mit dem im Schaft befindlichen Schiebeteil koppelbar ist. Im Einzelnen wird dabei der zweite Griff in die Einhängeausnehmung eingeschoben. Dabei wird der Bereich des zweiten Griffs, welcher die Öffnung aufweist, mit Hilfe des Durchgriffs in das Innere aus Schaft und Schiebeteil geschoben. Das Schiebeteil wiederum weist einen Pin auf, welcher in die Ausnehmung eingebracht werden kann.

In einem besonders bevorzugten Ausführungsbeispiel ist der Kraftspeicher ein endseitig an das Schiebeteil angeformter Bügel. Der Bügel steht mit einem Anschlag des Schafts in Wirkverbindung, wobei der Bügel den zweiten Griff von dem ersten Griff rückstellt. Form, Grösse und Ausbildung des Bügels sowie des Anschlags sind abhängig von dem Einsatzgebiet des chirurgischen Instrumentes sowie des genutzten Funktionselementes. So kann beispielsweise der Bügel auch als Halbkreisform oder mit Verstärkungsrippen oder dergleichen ausgeführt werden.

Zum Zerlegen des chirurgischen Instruments kann mit folgenden Schritten vorgegangen werden:
- das Schiebeteil wird seitlich von dem Schaft weg gedrückt,
- der zweite Griff wird aus dem Schaft gezogen und
- das freigegebene Schiebeteil wird aus dem Schaft entfernt.

Das erfindungsgemässe chirurgische Instrument wird u.a.. durch folgende Schritte hergestellt:
Öffnungen und Schlitze werden mittels eines Scheibenfräsers eingebracht, Hinterschneidungen und Nuten werden durch Umformen gebildet.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
Figur 1 zeigt eine Seitenansicht eines erfindungsgemässen Litzeldrahtschneiders;
Figur 2 zeigt eine Seitenansicht eines erfindungsgemässen Litzeldrahtschneiders;
Figur 3 zeigt eine Seitenansicht eines ersten Teils eines erfindungsgemässen Litzeldrahtschneiders;
Figur 4 zeigt eine Draufsicht des Teils nach Figur 3
Figur 5 zeigt eine Seitenansicht eines zweiten Teils eines erfindungsgemässen Litzeldrahtschneiders;
Figur 6 zeigt eine Draufsicht des Teils nach Figur 5;
Figur 7 zeigt eine Seitenansicht eines weiteren Teils eines erfindungsgemässen Litzeldrahtschneiders.

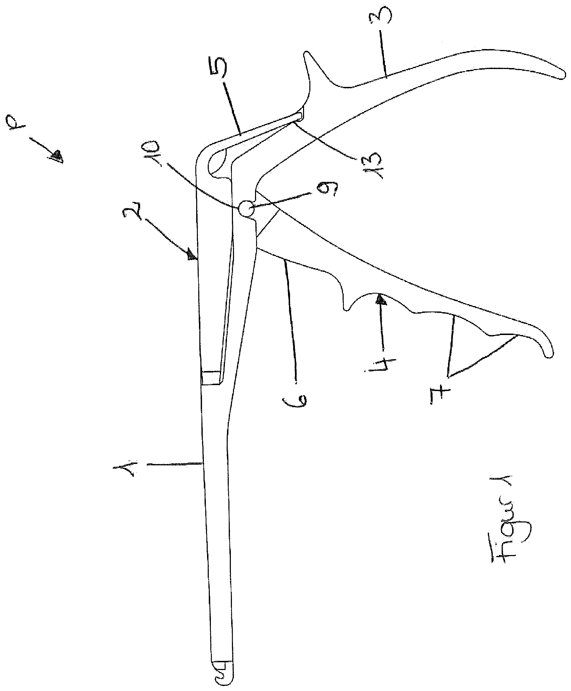

In Figur 1 ist ein Seitenansicht eines Litzendrahtschneiders P in Gebrauchslage dargestellt. Der Litzendrahtschneider P besteht aus einem Schaft 1, an den ein erster Griff 3 angeformt ist.

Weiterhin ist ein Schiebeteil 2 vorhanden. Das Schiebeteil 2 ist in Figur in den Schaft 1 eingeschoben dargestellt.

Daneben ist ein zweiter Griff 4 gezeigt. Einends weist der zweite Griff 4 einen Haltebereich 6 auf. Der Haltebereich 6 wiederum besteht teilweise Fingermulden 7.

An dem Schiebteil 2 ist einends einstückig ein Bügel 5 angeformt. Dabei ragt der Bügel 5 im wesentlichen rechtwinklig vom Schiebeteil 2 ab.

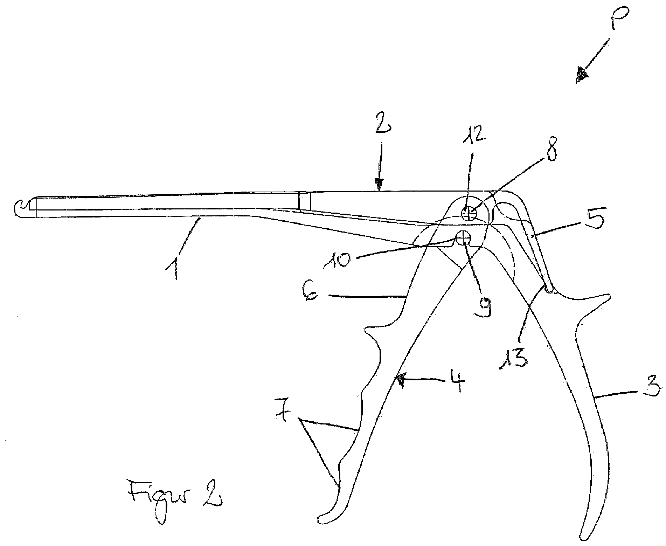

Der zweite Griff 4 weist andernends eine Ausnehmung 8 zur Koppelung mit dem Schiebeteil 2 auf, wobei die Ausnehmung 8 besser in Figur 2 zu erkennen ist.

Daneben weist der zweite Griff 4 einen Einhängebolzen 9 zur Koppelung mit dem Schaft 1 auf. Dabei wird der Einhängebolzen 9 in eine Einhängeausnehmung 10 des Schafts 1 eingeschoben dargestellt.

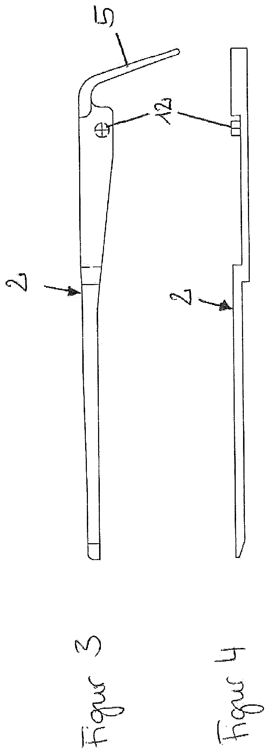

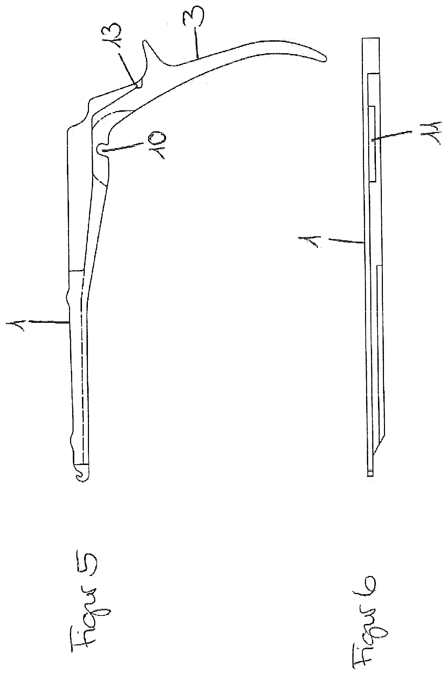

Im Bereich der Einhängeausnehmung 10 ist ein Durchgriff 11 in dem Schaft 1 vorhanden. Dieser Durchgriff 11 ist in Figur 6 besonders gut zu erkennen. Damit ragt der zweite Griff 4 derart in den Schaft 1 hinein, dass die Ausnehmung 8 mit dem im Schaft 1 befindlichen Schiebeteil 2 koppelbar ist. Die Kopplung des zweiten Griffs 4 erfolgt dadurch, dass das Schiebeteil 2 einen Pin 12 aufweist, welcher in die Ausnehmung 8 einbringbar ist. Die Ausformung des Pins 12 ist in den Figuren 3 und 4 besonders gut zu erkennen.

In Figur 3 ist das Schiebeteil 2 in einer Seitenansicht gezeigt. Dabei ist andernends zum Bügel 5 eine nicht weiter gekennzeichnete Schneide vorhanden. In Figur 4 ist gezeigt, dass das Schiebeteil 2 in Form eines Fragezeichens ausgeformt ist, wobei der Pin 12 in einem ausgenommenen Bereich des Schiebeteils 2 ausgeformt ist.

In Figur 5 ist eine Seitenansicht eines Schaft 1 gezeigt. Dort ist im Übergang zwischen dem Bereich der Einhängeausnehmung 10 und dem ersten Griff 3 ein

Anschlag 13 vorhanden. Der Anschlag 13 dient als Kraftanker zu dem Bügel 5 des Schiebeteils 2.

In Figur 6 ist neben dem im Bereich der Einhängeausnehmung 10 vorhandenen Durchgriff 11 auch zu erkennen, wie der Schaft 1 etwa auf einem Drittel seiner Fläche als U-Profil ausgeformt ist. Die Ausformung eines U-Profil dient der besseren verschiebbaren und doch haltenden Lagerung des Schiebeteils 2.

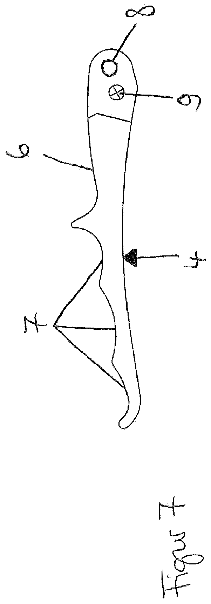

In Figur 7 ist zu erkennen, wie an dem zweiten Griff 4 die endseitig eingebrachte Öffnung 8 ausgeformt ist. Von der Öffnung 8 zu den Fingermulden 7 hin ist dann der Einhängebolzen 9 nochmals gezeigt.

**Bezugszeichenliste**

| | | | | | |
|---|---|---|---|---|---|
| 1 | Schaft | 34 | | 67 | |
| 2 | Schiebeteil | 35 | | 68 | |
| 3 | Erster Griff | 36 | | 69 | |
| 4 | Zweiter Griff | 37 | | 70 | |
| 5 | Bügel | 38 | | 71 | |
| 6 | Haltebereich | 39 | | 72 | |
| 7 | Fingermulden | 40 | | 73 | |
| 8 | Ausnehmung | 41 | | 74 | |
| 9 | Einhängebolzen | 42 | | 75 | |
| 10 | Einhängeausnehmung | 43 | | 76 | |
| 11 | Durchgriff | 44 | | 77 | |
| 12 | Pin | 45 | | 78 | |
| 13 | Anschlag | 46 | | 79 | |
| 14 | | 47 | | | |
| 15 | | 48 | | | |
| 16 | | 49 | | | |
| 17 | | 50 | | | |
| 18 | | 51 | | | |
| 19 | | 52 | | | |
| 20 | | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Chirurgisches Instrument mit
- einem Schaft (1), an dem ein erster Griff (3) ausgeformt ist;
- einem Schiebeteil (2), welcher durch einen zweiten Griff (4) betätigbar ist und
- einem Kraftspeicher zum Rückstellen des Schiebeteils (2),
**dadurch gekennzeichnet,**
**dass** der Kraftspeicher und das Schiebeteil (2) einstückig ausgebildet sind,
- wobei der Kraftspeicher ein endseitig an das Schiebeteil (2) oder den Schaft (1) oder den zweiten Griff (4) angeformter Bügel (5) ist,
- wobei im Bereich einer Einhängeausnehmung (10) des Schaftes (1) zur Aufnahme eines Einhängebolzens (9) ein Durchgriff (11) in dem Schaft (1) vorhanden ist, damit der zweite Griff (4) derart in den Schaft (1) hineinragt, dass die Ausnehmung (8) mit dem im Schaft (1) befindlichen Schiebeteil (2) koppelbar ist,
- wobei der Bügel (5) mit einem Anschlag (13) des Schafts (1) in Wirkverbindung steht, wobei der Bügel (5) den zweiten Griff (4) von dem ersten Griff (3) rückstellt.

2. Chirurgisches Instrument nach Anspruch 1, **dadurch gekennzeichnet, dass** der zweite Griff (4) einends einen Haltebereich (6) aufweist.

3. Chirurgisches Instrument nach Anspruch 2, **dadurch gekennzeichnet, dass** der Haltebereich (6) Fingermulden (7) umfasst.

4. Chirurgisches Instrument nach einem der vorigen Ansprüche, **dadurch gekennzeichnet, dass** der zweite Griff (4) andernends eine Ausnehmung (8) zur Koppelung mit dem Schiebeteil (2) aufweist.

5. Chirurgisches Instrument nach einem der vorherigen Ansprüche, **dadurch gekennzeichnet, dass** der zweite Griff (4) einen Einhängebolzen (9) zur Koppelung mit dem Schaft (1) ausbildet.

6. Chirurgisches Instrument nach Anspruch 5, **dadurch gekennzeichnet, dass** der Einhängebolzen (9) unrund ausgeformt ist.

7. Chirurgisches Instrument nach Anspruch 5 oder 6, **dadurch gekennzeichnet, dass** der Schaft (1) eine Einhängeausnehmung (10) zur Aufnahme des Einhängebolzens (9) aufweist.

8. Chirurgisches Instrument nach Anspruch 4, **dadurch gekennzeichnet, dass** das Schiebeteil (2) einen Pin (12) aufweist, welcher in die Ausnehmung (8) einbringbar ist.

9. Verfahren zum Zerlegen des chirurgischen Instruments nach einem der Ansprüche 1 bis 8 **gekennzeichnet durch** folgende Schritte:
- das Schiebeteil (2) wird seitlich von dem Schaft (1) weg gedrückt,
- der zweite Griff (4) wird aus dem Schaft (1) gezogen und
- das freigegebene Schiebeteil (2) wird aus dem Schaft (1) entfernt.

## Claims

1. Surgical instrument having
- a shaft (1) on which a first handle (3) is formed;
- a sliding part (2) which can be actuated by a second handle (4) and
- a force accumulator for returning the sliding part (2),
**characterised**
**in that** the force accumulator and the sliding part (2) are formed in one piece,
- wherein the force accumulator is a bow (5) formed integrally at the end on the sliding part (2) or the shaft (1) or the second handle (4),
- wherein in the area of a hook-in recess (10) of the shaft (1), a passage (11) is present in the shaft (1) for receiving a hook-in bolt (9), such that the second handle (4) protrudes into the shaft (1) in such a way that the recess (8) can be coupled to the sliding part (2) located in the shaft (1),
- wherein the bow (5) is operatively connected to a limit stop (13) of the shaft (1), wherein the bow (5) returns the second handle (4) from the first handle (3).

2. Surgical instrument according to claim 1, **characterised in that** the second handle (4) has a holding area (6) at one end.

3. Surgical instrument according to claim 2, **characterised in that** the holding area (6) comprises finger depressions (7).

4. Surgical instrument according to one of the preceding claims, **characterised in that** the second handle (4) has, at the other end, a recess (8) for coupling to the sliding part (2) .

5. Surgical instrument according to one of the preceding claims, **characterised in that** the second handle (4) forms a hook-in bolt (9) for coupling to the shaft (1).

6. Surgical instrument according to claim 5, **characterised in that** the hook-in bolt (9) is of non-circular shape.

7. Surgical instrument according to claim 5 or 6, **characterised in that** the shaft (1) has a hook-in recess (10) for receiving the hook-in bolt (9).

8. Surgical instrument according to claim 4, **characterised in that** the sliding part (2) has a pin (12) which can be introduced into the recess (8).

9. Method for dismantling the surgical instrument according to one of claims 1 to 8 **characterised by** the following steps:
- the sliding part (2) is pressed laterally away from the shaft (1),
- the second handle (4) is pulled from the shaft (1) and
- the released sliding part (2) is removed from the shaft (1).

## Revendications

1. Instrument chirurgical avec
- un manche (1) sur lequel est formée une première poignée (3);
- un élément coulissant (2) qui peut être actionné par une deuxième poignée (4), et
- un accumulateur d'énergie pour retourner l'élément coulissant (2),
**caractérisé par le fait que** l'accumulateur d'énergie et l'élément coulissant (2) sont réalisés de manière solidaire,
- dans lequel l'accumulateur d'énergie est une bride (5) formée du côté de l'extrémité sur l'élément coulissant (2) ou le manche (1) ou la deuxième poignée (4),
- dans lequel est prévu, à l'endroit d'un évidement d'accrochage (10) du manche (1) destiné à recevoir un boulon d'accrochage (9), un passage (11) pour que la deuxième poignée (4) pénètre dans le manche (1) de sorte que l'évidement (8) puisse être couplé à l'élément coulissant (2) se trouvant dans le manche (1),
- dans lequel la bride (5) est en communication active avec une butée (13) du manche (1), la bride (5) retournant la deuxième poignée (4) depuis la première poignée (3).

2. Instrument chirurgical selon la revendication 1, **caractérisé par le fait que** la deuxième poignée (4) présente à une extrémité une zone de saisie (6).

3. Instrument chirurgical selon la revendication 2, **caractérisé par le fait que** la zone de saisie (6) comporte des creux pour les doigts (7).

4. Instrument chirurgical selon l'une des revendications précédentes, **caractérisé par le fait que** la deuxième poignée (4) présente à l'autre extrémité un évidement (8) pour le couplage avec l'élément coulissant (2).

5. Instrument chirurgical selon l'une des revendications précédentes, **caractérisé par le fait que** la deuxième poignée (4) forme un boulon d'accrochage (9) pour le couplage avec le manche (1).

6. Instrument chirurgical selon la revendication 5, **caractérisé par le fait que** le boulon d'accrochage (9) est réalisé de forme non ronde.

7. Instrument chirurgical selon la revendication 5 ou 6, **caractérisé par le fait que** le manche (1) présente un évidement d'accrochage (10) destiné à recevoir le boulon d'accrochage (9).

8. Instrument chirurgical selon la revendication 4, **caractérisé par le fait que** l'élément coulissant (2) présente une broche (12) qui peut être introduite dans l'évidement (8).

9. Procédé de démontage de l'instrument chirurgical selon l'une des revendications 1 à 8, **caractérisé par** les étapes suivantes:
- l'élément coulissant (2) est poussé latéralement pour s'éloigner du manche (1),
- la deuxième poignée (4) est retirée du manche (1), et
- l'élément coulissant (2) libéré est sorti du manche (1).
